# EUROPEAN PATENT APPLICATION

(11) **EP 3 771 442 A1**
(43) Date of publication of application: **03.02.2021**
(21) Application number: 19305992.0
(22) Date of filing: 31.07.2019
(51) Int. Cl.: A61B 17/16, A61B 17/00

(54) **MODULAR TOOL**

(71) Applicant: Ostium Group, 44360 Saint Etienne De Montluc (FR)
(72) Inventor: BIBARD, Léopold, 44360 SAINT ETIENNE DE MONTLUC (FR); SOQUENNE, Edgard, 44360 SAINT ETIENNE DE MONTLUC (FR); RETAILLEAU, Vincent, 44360 SAINT ETIENNE DE MONTLUC (FR)
(74) Representative: Marks & Clerk France

(57) **Abstract**

A modular surgical tool (300, 1200), for example for hip, shoulder, knee or bone traumatology is provided comprising a handle (310), and interface part (330) and a tool (350). The interface may serve to provide a selected linear and/or angular offset with regard to the respective axes of the handle and tool. The handle may be releasably coupled to the interface element by a threaded ring (420) on a threaded cylindrical member (411) of the handle. The handle may comprise a slot (413) in which a keyed element (406) of the interface may engage, to be trapped when the ring is turned about the cylindrical member. Meanwhile the interface may be releasably coupled to the tool by means of a keyed element (841) of the tool which may be slid into a slot (820) of the interface distal end, the slot being intersected by a securing pin channel (830) passing through the body of the interface from the other side, such that when the securing pin (850) is inserted, the keyed element of the tool is trapped in the slot.

## Description

### Field of the invention

The present invention relates to modular tools.

### Background of the invention

The concept of modularity in tools is almost as old as tool use itself. Many ancient tool designs such as hammers, axes, picks and the like comprise a working element fixed to a handle by some means which allows the periodic replacement of one element or the other. Developments in manufacturing processes over the last two hundred years meanwhile have made it possible to develop releasable fixing mechanisms. For example, screw driver heads are commonly available as 6.35mm (quarter inch) hexagon bits, which may be inserted as required in a standard handle. The bits may be secured magnetically, or by means of a spring loaded ball bearing engaging a groove in the bit. In some contexts meanwhile, the operational constraints extant in that context may tend to lead away from such approaches. In the field of surgical instruments for example, the high forces involved along with sterilisation requirements may tend to indicate an all-in-one approach.

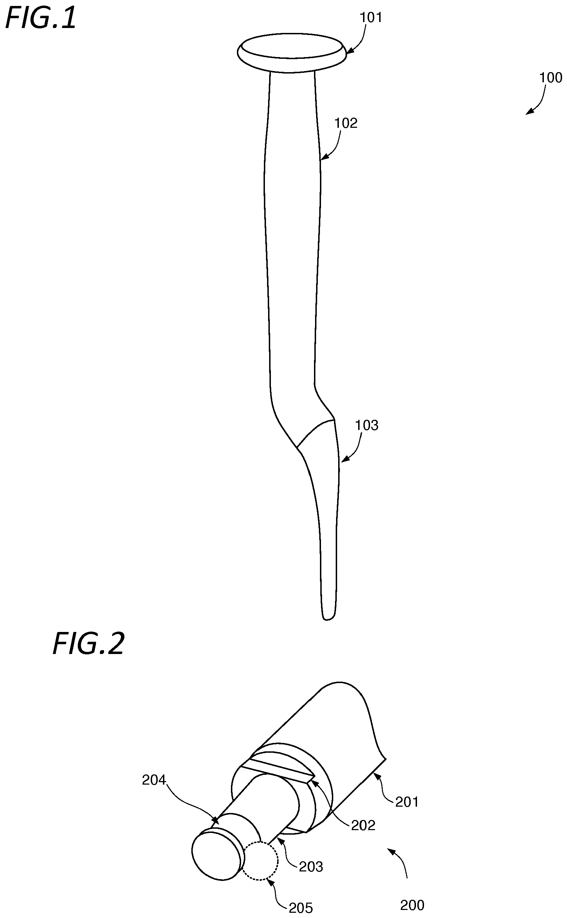

Figure 1 shows an orthopaedic reamer as known in the state of the art.

As shown in figure 1, a reamer 100 comprises a handle 102 with a strike head 101 and a reamer working surface 103. The reamer 100 is typically made entirely of stainless steel.

Notwithstanding the foregoing, the cost of manufacturing a complete set of stand-alone tools for example as shown in figure 1 can be considerable, and even in the field of surgical tools, some attempts at modular tools are known.

In this, as in any field where a solid positive engagement between the handle and further elements is a critical requirement, special consideration must be given to the securing mechanism.

Figure 2 shows a releasable securing mechanism for modular tools as known in the state of the art.

Figure 2 shows a conventional securing mechanism known in the art as a "Hudson Fitting". In particular, figure 2 shows a male Hudson fitting 200, attached to a tool element 201. The fitting comprises a cylindrical member 203 with a semi-circular channel or groove 204. When the cylindrical member 203 is slid into the corresponding female element, a spring loaded ball bearing 205 engages the channel so as to prevent accidental decoupling. In implementations where uncoupling must be avoided in the presence of a separating force, the ball bearing may be replaced with a removable cotter pin or the like. As shown, the fitting also comprises a flattened flange 202 at the proximal end of the fitting closest to the tool element 201. The flats of this flange may engage corresponding surfaces on the corresponding female element when the coupling is fully inserted, so that rotational forces may be effectively transferred between the two elements of the coupling.

It is desirable to provide a flexible solution for surgical instruments.

### Summary of the invention

In accordance with the present invention in a first aspect there is provided a modular instrument for surgery of the hip, shoulder, knee or bone traumatology, the instrument comprising a handle part, an interface part and a tool element, the handle part being releasably coupled to the interface part and the interface part being releasably coupled to the tool part.

In a development of the first aspect, the handle and the tool each has a respective primary axis, whereby the interface part is configured to establish a lateral offset between the axes.

In a development of the first aspect, the handle and the tool each has a respective primary axis, whereby the interface part is configured to establish an angular offset between the axes.

In a development of the first aspect, the handle part comprises a body having a distal end and a proximal end, the distal end providing a releasable coupling for an element of the modular tool. The releasable coupling comprises a cylindrical member provided with a first external helical thread and a first lateral slot, the first slot opening on one periphery of the distal end of the body, the first slot widening from the distal end towards the proximal end. The releasable coupling further comprises a threaded ring, the threads of the ring engaging the first external helical thread of the cylindrical member. The threaded ring is rotatable about the distal end of the member between an extended position in which the ring obstructs the first slot opening on one periphery of the distal end of the body, and a retracted position in which the ring leaves the first slot opening on one periphery of the distal end of the body unobstructed.

In a development of the first aspect, the modular instrument comprises the angular displacement of the threaded ring when rotated between the extended position and the retracted position is between 89 and 181 degrees.

In a development of the first aspect, the threaded ring comprises a detent at the distal edge thereof, the detent being positioned on the circumference of the ring such that when the ring is in the retracted position the indentation is aligned with the first slot.

In a development of the first aspect, the interface part has a distal end and a proximal end disposed on a longitudinal axis, the interface part comprising a second lateral slot disposed along a second axis, the second axis in the plane of the first axis and being at an angle of between 10 and 80 degrees to the longitudinal axis. The second slot widens from top to bottom to an entry aperture at the distal end of the second slot. The interface part further comprises a securing pin channel extending through the interface part and terminating in the entry aperture, such that a securing pin may be inserted through the pin channel so as to trap a keyed element inserted in the widened part of the second slot, whereby the interface part may be releasably coupled to the tool part by means of a keyed element of the tool engaged in the second slot and trapped by the securing pin.

In a development of the first aspect, the securing pin channel extends along a third axis, the third axis being at an angle in the plane of the first and second axes of between 90 degrees to the second axis and 30 degrees to the second axis in the proximal direction.

In a development of the first aspect, the dimensions of the lateral slot vary along the second axis so that the force required to slide a corresponding keyed element of the tool into the lateral slot increases as the corresponding keyed element of the tool progresses into the slot.

In a development of the first aspect, the modular tool comprises a surgical instrument.

In a development of the first aspect, the modular tool is for surgery of the hip, shoulder, knee or bone traumatology.

In a development of the first aspect, the modular tool comprises a rasp or reamer.

In a development of the first aspect, at least a part of the modular instrument is composed of a synthetic material or a synthetic composite material.

In a development of the first aspect, at least a part of the modular instrument is composed of a glass fiber reinforced polyarylamide.

### Brief Description of the Drawings

The above and other advantages of the present invention will now be described with reference to the accompanying drawings, for illustration purposes only, in which:
Figure 1 shows an orthopaedic reamer as known in the state of the art;
Figure 2 shows a releasable securing mechanism for modular tools as known in the state of the art;
Figure 3 shows a modular instrument for surgery of the hip, shoulder, knee or bone traumatology;
Figure 4 shows details of a first releasable coupling in accordance with certain embodiments;
Figure 5a shows the handle in a first, retracted position prior to the insertion of the secondary element 405. The secondary element may comprise a tool 350 as described above;
Figure 5b shows the handle in a first, retracted position after insertion of the secondary element 405;
Figure 5c shows the handle in a second, extended position after insertion of the secondary element 405;
Figure 6a shows a sectional view of the slot in a first embodiment;
Figure 6b shows a sectional view of the slot in a second embodiment;
Figure 7 shows a plan view of a keyed element corresponding to the slot of figure 6a;
Figure 8a shows an interface part for a modular tool in accordance with an embodiment in a first configuration;
Figure 8b shows an interface part for a modular tool in accordance with an embodiment in a second configuration;
Figure 8c shows an interface part for a modular tool in accordance with an embodiment in a third configuration;
Figure 8d shows an interface part for a modular tool in accordance with an embodiment in a fourth configuration;
Figure 9 shows an interface element for a modular tool in accordance with a further embodiment;
Figure 10a shows a tool and interface part in accordance with an embodiment in a first configuration;
Figure 10b shows a tool and interface part in accordance with an embodiment in a second configuration;
Figure 10c shows a tool and interface part in accordance with an embodiment in a third configuration;
Figure 10d shows a tool and interface part in accordance with an embodiment in a fourth configuration; and
Figure 11 shows a tool in accordance with an embodiment.

### Detailed description

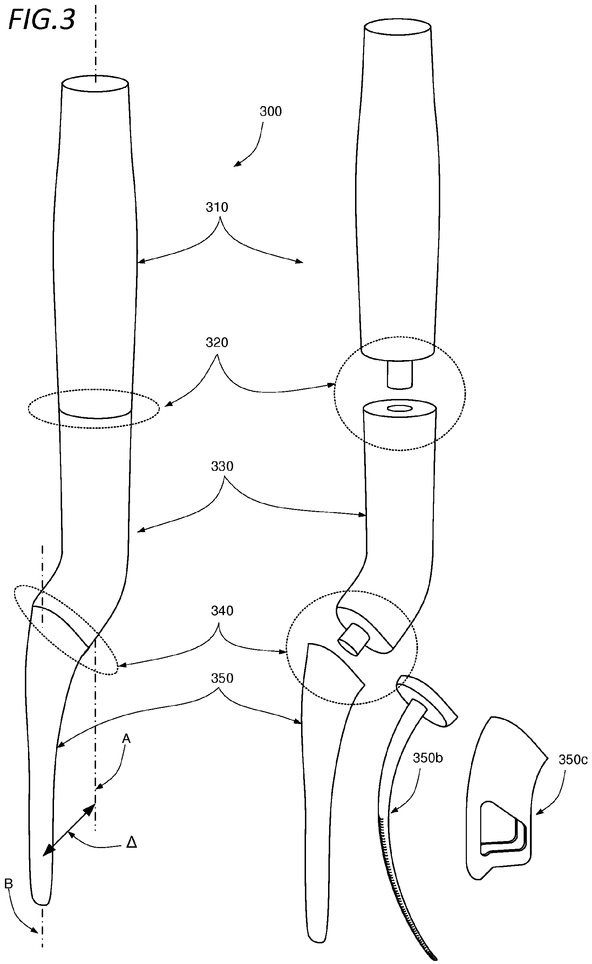

Figure 3 shows a modular instrument for surgery of the hip, shoulder, knee or bone trau matology.

As shown, the instrument 300 of figure 3 comprises a handle part 310, an interface part 330 and a tool element 350. The handle part 310 is releasably coupled to the interface part 330 and the interface part 330 is releasably coupled to the tool part 350. Accordingly, while two part modular tools may be known in the state of the art, the present invention provides a tool with three parts.

By way of example, the instrument 300 of figure 3 comprises a first releasable coupling 320 between the handle part 310 and interface part 330, and a second releasable coupling 340 between interface part 330 and the tool part 350.

Either or both of the first releasable coupling 320 and second releasable coupling 340 may comprise Hudson fittings for example as described above, or threaded fittings, or any other type of fitting as may be known in the state of the art and deemed appropriate to a particular implementation.

As shown in figure 3, the handle 310 has a primary axis A, and the tool 350 has a primary axis B, whereby the interface part 330 is configured to establish a lateral offset Δ between these axes. This arrangement may be particularly desirable in the context of certain types of instrument for surgery of the hip, shoulder, knee or bone traumatology where it is required to bring significant forces to bear in constrained spaces, and at an angle dictated by the patient's anatomy.

The interface part 330 may also or alternatively be configured to establish an angular offset between the axes A and B. This arrangement may be particularly desirable in the context of certain types of instrument for surgery of the hip, shoulder, knee or bone traumatology where it is required to bring significant forces to bear in constrained spaces, and at an angle dictated by the patient's anatomy.

A tool set in accordance with an embodiment may comprise at least one handle, one or more different tool elements, and a plurality of interface elements, each said interface element defining a different combination of angular and/or linear offsets with regard to the primary axes of the handle and tool when coupled to each respective said interface.

The tool 350 may comprise a surgical instrument. More particularly for example, the tool may be for surgery of the orthopaedic surgery or bone traumatology. Still more particularly for example tool may comprise a rasp or reamer or impactor. Alternatively, the secondary element may comprise an intermediate adaptor between the handle and a further component, where the further component may comprise a tool as discussed above or otherwise.

One field in which a handle as described may be appropriate is that of surgical instruments, such that the modular tool as a whole may comprise or constitute a surgical instrument. More particularly, the modular tool may be for orthopaedic surgery or bone traumatology. More particularly, the modular tool may be for surgery of the hip, shoulder or knee. More particularly, as shown, the working tool 350 and thus the modular tool as a whole 300 comprises a rasp or reamer or impactor. It will be appreciated that in line with the many fields of application and associated tool types that may be envisaged, many different possible working parts 350 may be envisaged, for use with a single handle in accordance with embodiments as described herein. Further examples of possible working parts, and resulting modular tools, include a curved rasp 350b, osteotome 350c and many other tools as will readily occur to the skilled person.

The handle may optionally be provided with an angle datum such as a radial line on the guard plate, or a radial lumen through which a bar may by inserted.

The handle, the interface part and the tool may each be formed of any material. In particular, it may be formed of steel, aluminium, titanium or any other suitable metal or alloy. It may also be formed of a thermoplastic or other synthetic material. It may in particular be formed from a polyamide, for example a polyarylamide. The synthetic material may comprise additional components such as a filler, swelling agent and the like. It may still further be formed of a synthetic composite material, comprising a glass, carbon fibre, carbon nanoparticle or any other material exhibiting a high tensile strength, in a matrix of a synthetic material, such as any of those listed above. In certain embodiments, the interface part may be composed of a glass fibre reinforced polyarylamide, such as for example that marketed by the Solvay corporation under the trademark "Ixef 1022" or more particular "Ixef GS 1022".

The handle, the interface part and the tool may be formed of different respective materials, and different regions of some or each of the handle, the interface part and the tool may be formed of different including metal parts and synthetic parts. The handle may also comprise voids for the purpose of economy of material, reduced weight and so on.

In some embodiments, the handle, interface part and working part may all be composed of the same material. In certain embodiments, the handle, interface part and working part may all be formed of a glass fibre reinforced polyarylamide, for example that marketed by the Solvay corporation under the trademark "Ixef 1022".

While as discussed above either or both of the releasable couplings 320 and 340 may comprise conventional releasable couplings, there will now be described a novel type of releasable coupling particularly adapted to the present context of the first releasable coupling 320.

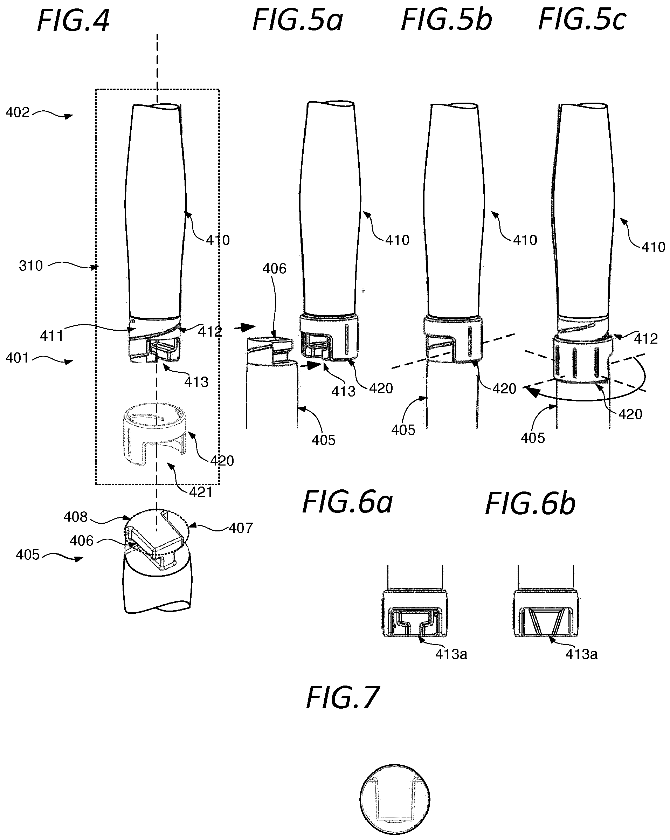

Figure 4 shows details of a first releasable coupling in accordance with certain embodiments.

A shown in figure 4, the handle part 310 may comprise a body 410 having a distal end 401 and a proximal end 402, the distal end providing a releasable coupling 320 for an element of the modular tool. As shown, the releasable coupling comprises a cylindrical member 411 provided with a first external helical thread 412, and a first lateral slot 413. The first slot opens on one periphery of the distal end of the body 410, the first slot 413 widening from the distal end towards the proximal end. The releasable coupling further comprises a threaded ring 420, the threads of this ring 420 engage the first external helical thread 412 of the cylindrical member 411. The threaded ring 420 is rotatable about the distal end of the member between an extended position in which the ring obstructs the first slot 413 opening on one periphery of the distal end of the body 410, and a retracted position in which the ring leaves the first slot opening 413 on one periphery of the distal end of the body 410 unobstructed.

Where a plurality of helical threads are provided, one or more of these threads may have dimensions different to other threads, such that the ring can only engage the cylindrical member in the desired orientation, for example so as to ensure proper positioning of the optional detent with respect to the slot in the extended and retracted positions. For example, the helical thread has a first root to crest ratio, and the second helical thread has a second root to crest ratio, wherein the threads of the ring are defined so that the ring can only engage the corresponding respective helical thread, and thereby may only be mounted on the handle in one configuration.

Similarly, the circumferential spacing of the threads may be varied to achieve the same effect.

The threads on the cylindrical member and/or the ring need not be continuous.

As shown, the ring is provided with an optional detent 421, in the distal circumferential edge thereof.

It will be appreciated that there is provided a secondary element having features in correspondence to the handle of the present invention, and in particular to the releasable coupling thereof. In particular, there is provided a secondary element for a modular tool, the secondary element having a distal end and a proximal end disposed on a longitudinal axis, the distal end of said secondary element being enclosed within a notional cylinder 407. The secondary element comprises a first keyed element 406 widening in a distal direction. A trailing edge 408 of the keyed element conforms to the cylinder 407, whereby said secondary element may be secured in a corresponding handle by inserting said first keyed element 406 with the trailing edge 408 outward into a corresponding slot defined in said handle, whereby a ring 420 of said handle corresponding to said cylinder may be slid into position so as to block said keyed element 406 in said handle.

The trailing edge 408 of the keyed element which conforms to the cylinder may furthermore be partially threaded so as to provide a continuous thread with threads provided in a cylindrical member of the handle, and adapted to engage threads of the ring as described above.

Figures 5a, 5b and 5c show the handle of figure 4 in different configurations.

As shown, the threaded ring 420 is rotatable about the distal end 401 of the member 411 from a first, retracted position as shown in figures 4a and 4b, to a second, extended as shown in figure 4c.

Figure 5a shows the handle in a first, retracted position prior to the insertion of the secondary element 405. The secondary element may comprise a tool 350 as described above.

As shown, the secondary element 405 comprises a tongue or keyed element 406 which has a form complementary to the slot 413. As shown the ring 420 is in a retracted position in which the ring leaves the slot opening on one periphery of the distal end of the body unobstructed. By this means, the keyed element 406 of the secondary element 405 may be introduced into the slot 413 of the handle.

As shown, the optional detent 421 aligns with the slot in the retracted position.

Figure 5b shows the handle in a first, retracted position after insertion of the secondary element 405.

Since the slot 413 widens from the distal end 401 towards the proximal end 402, a correspondingly formed keyed element 406 may be slid into the slot laterally as shown in figure 5b, but once in position, will not be movable along the axis of the handle, i.e. towards or away from the distal end of the handle, but only back or forth along the axis of the slot.

Figure 5c shows the handle in a second, extended position after insertion of the secondary element 405. As shown in figure 5c, the ring has been rotated about the cylindrical member 411, so that through the engagement of the threads of the ring in the threads 412 of the cylindrical member, this rotational movement has been translated into a linear movement towards the distal end of the handle, so that the ring obstructs the slot opening on one periphery of the distal end of the body.

As shown, this movement comprises a rotation of 180° clockwise about the axis of the handle when viewed down the length from the proximal to the distal end of the handle. The skilled person will appreciate that the pitch of the threads and the angle through which the ring is rotated will determine the distance along the cylindrical member that the ring 420 travels. On the one hand it is desirable that the distance travelled should be as great as possible, to allow the use of a long keyed element 406, providing a strong and rigid connection between the handle and secondary element. On the other hand, in use it will be desirable that the user be required to turn the circle through as small an angle as possible, so that the manipulation may be performed with the lowest possible demand on the user's dexterity. On this basis, the angular displacement of the ring between the extended position and the retracted position is preferably less than 361°, more preferably less than 271°, more preferably less than 181°. A multiple of 90° may be advantageous in terms of being more intuitive to the user. The angular displacement of the ring between the extended position and the retracted position is preferably more than 89°. The chosen compromise between these considerations is defined by the pitch of the respective threads of the ring and the cylindrical member. Additional constraints may occur through a desire to limit the likelihood of the ring moving along the cylindrical member under its own weight, or in response to an accidental or incidental pressure, which will tend to favour a tighter pitch, although these considerations may also be managed by selecting materials, surfaces and/or tolerances so that the friction between elements reduces the risk of such unwanted movement. On this basis, the threads of the ring and cylindrical member may be configured in accordance with an embodiment such that the ring is turned through substantially one single revolution or less between the extended position and the retracted position. In accordance with a further embodiment the ring is turned through substantially one half revolution or less between the extended position and the retracted position. In accordance with a further embodiment the ring is turned through substantially one quarter revolution or less between the extended position and the retracted position. In accordance with a further embodiment the ring is turned through substantially one third revolution or more between the extended position and the retracted position. Meanwhile, the threads of the ring and cylindrical member may be configured in accordance with an embodiment such that the ring moves along the axis of the cylindrical member a distance substantially equal to 2 cm or less between the extended position and the retracted position. In accordance with a further embodiment the ring moves along the axis of the cylindrical member a distance substantially equal to 1,5 cm or less between the extended position and the retracted position. In accordance with a further embodiment the ring moves along the axis of the cylindrical member a distance substantially equal to 1 cm or less between the extended position and the retracted position. The skilled person will appreciate that any combination of angle of rotation and linear displacement may be selected within these ranges, and indeed outside these ranges. It may be noted that the thread of the cylindrical member and ring of figures 5a, 5b and 5c comprises a double thread. A double thread may be advantageous in providing a firm engagement between the threads of the ring and the cylinder while permitting a short threaded section along the length of the ring, so as to minimise the dimensions of the ring. The skilled person will appreciate that a single thread may also be adequate in many implementations. The skilled person will appreciate that three or even more threads may be appropriate in other implementations.

As shown in figures 4 and 5a, the slot 413 defines substantially a T cross section, and the keyed element 406 is shaped correspondingly. This shape achieves the objective of ensuring that the keyed element can only enter or exit the slot through the lateral opening of the cylindrical section, and that once the ring is in the extended position, no force in any direction on the secondary element with respect to the handle will separate the secondary element from the handle. The skilled person will recognise that the keyed element and corresponding slot may have any form complying with the general requirement that it widens from the distal end towards the proximal end. As such, it may form a wedge, dovetail or T section as described above. It may furthermore be circular, elliptical, rectangular, square, or any other form. The keyed element will generally constitute an extrusion of the chosen cross section from one side to the other. In certain embodiments, the keyed element may taper from one side to the other. Where this is the case the slot may taper from side to side correspondingly. Where this is the case, the slot may be defined as being deeper from side to side that the length from side to side of the corresponding keyed element. On this basis, the tapering walls of the keyed element will engage the sides of the keyed element before the end of the keyed element reached the lateral extremity of the slot. By this means, the slot will become progressively tighter as the keyed element is inserted, and a firm insertion without any play between the handle and secondary element may be achieved by pushing the keyed element fully into the slot.

Figure 5c shows the handle in a second, extended position after insertion of the secondary element 405.

As shown, the ring 420 has been rotated through 180° clockwise about the cylindrical member 411, and by the action of the engagement between the threads 412 on the cylindrical member 411 with those of the ring, the ring has progressed along the length of the cylindrical member so as to block the slot 413. It may further be noted that while in the retracted position as shown in figures 4a and 4b the optional detent 421 aligns with the slot, in the extended position of figure 4c the optional detent 421 no longer aligns with the slot. As such, the provision of the slot 421 means that the slot can be effectively blocked by a smaller linear movement of the ring.

As such, as shown the keyed element of the secondary element 405 is entirely trapped in the slot by the new position of the ring, as the result of a simple half turn (in the present example) of the ring, which may be effectuated by the user with a movement of the thumb, retaining the tool in the other fingers of the actuating hand, and leaving the other hand free. To further facilitate this action, the ring may be knurled, grooved, provided with a non-slip coating, provided with flats or otherwise treated to improve the users grip thereon. Where the ring is provided with flats, these may be even in number, and may further be dimensioned so that they may be engaged using a spanner of standard dimensions, for example as defined in ISO/TC 29/SC 10 and the like.

In certain embodiments, the shape or dimension of ring and cylindrical member, and in particular the thread on either or both of the ring or the cylindrical member may vary along their length, for example such that their engagement becomes progressively tighter towards the extended position, so that while it may move freely at certain portions of its travel, as it approaches the extended position it becomes somewhat resistant to movement. This approach may be advantageous in further reducing the risk of the ring being moved from the extended position inadvertently, which might otherwise lead to a loosening or even decoupling of the secondary element from the handle. Similarly, the engagement between the ring and cylindrical member may become progressively tighter towards the retracted position, so that while it may move freely at certain portions of its travel, as it approaches the retracted position it becomes somewhat resistant to movement. This approach may be advantageous in further reducing the risk of the ring being moved from the retracted position inadvertently, which might otherwise complicate the task of inserting the keyed element of the secondary element. The variations in shape or dimensions may include for example variations in the pitch, the minor diameter, the major diameter, or the root to crest ratio or any combination of these. Additionally or alternatively a similar effect may be achieved by a variation in the outer diameter of the cylindrical member and/or the inner diameter of the ring along the length thereof, for example so that as the ring moves towards the extended position, it becomes progressively tighter on the cylindrical member and/or so that as the ring moves towards the retracted position, it becomes progressively tighter on the cylindrical member.

As discussed above, the slot (and correspondingly the keyed element) widens from the distal end towards the proximal end. As discussed above, the slot, and correspondingly the keyed element may take many forms whilst satisfying this requirement. Figures 6a and 6b show two possible such implementations.

Figure 6a shows a sectional view of the slot in a first embodiment.

As shown in figure 6a, the slot 413a is generally T shaped in cross section. It may be noted that the shoulders defining the top of the narrow stem of the T shape are not entirely parallel with the flat top of the T shape. In other embodiments, these shoulders may be parallel with the top, or adopt any other angle as may be found to be expedient.

Figure 6b shows a sectional view of the slot in a second embodiment.

As shown in figure 6b, the slot 413b is generally in the form of a dovetail in cross section.

As shown in both figures 6a and 6b, the dimensions of the lateral slot vary radially (that is, from the edge towards the middle) so that the force required to slide a corresponding keyed element of the tool into the lateral slot increases as the corresponding keyed element of the tool progresses into the slot.

Figure 7 shows a plan view of a keyed element corresponding to the slot of figure 6a.

The keyed element of figure 7 corresponds to that of the secondary element 405 shown in figure 4. As shown, the slight tapering of the keyed element is visible.

While as discussed above either or both of the releasable couplings 320 and 340 may comprise conventional releasable couplings, there will now be described a novel type of releasable coupling particularly adapted to the present context of the second releasable coupling 340.

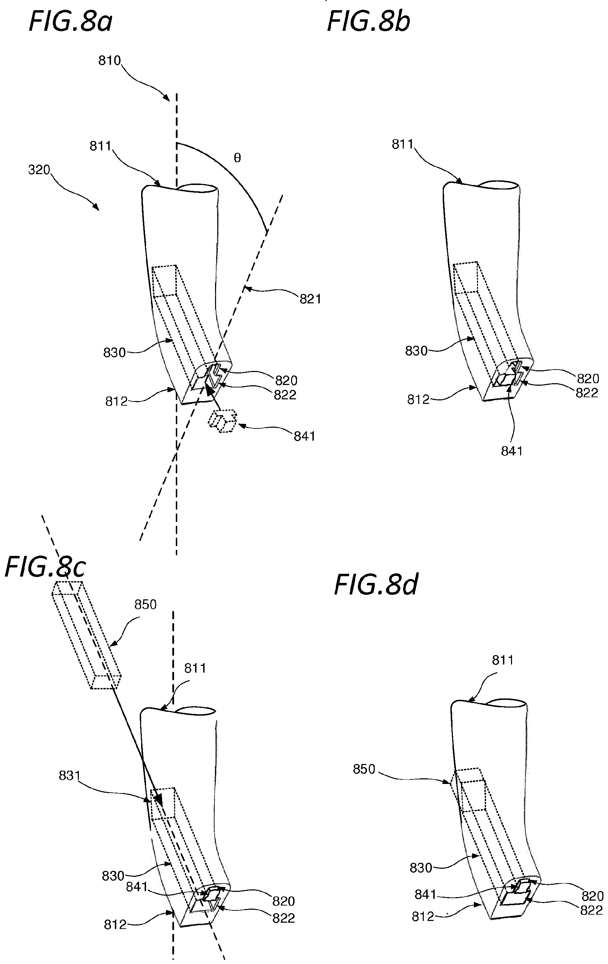

Figure 8a shows an interface part for a modular tool in accordance with an embodiment in a first configuration.

As shown, the interface part 320 has a distal end 812 and a proximal 811 end disposed on a longitudinal axis 810. The interface part 320 comprises a second lateral slot 820 disposed along a second axis 821, the second axis 821 in the plane of the first axis 810 and being at an angle θ of between 10 and 80 degrees to the longitudinal axis. The second slot 820 widens from top to bottom, to an entry aperture 822 at the distal end of the second slot 820. The interface part further comprises a securing pin channel 830 extending though the interface part and terminating in the entry aperture, such that a securing pin 850 may be inserted through the pin channel so as to trap a keyed element 841 inserted in the widened part of the second slot 820, whereby the interface part 330 may be releasably coupled to the tool part 350 by means of a keyed element 841 of the tool 350 engaged in the second slot 820 and trapped by the securing pin 850.

As shown in figure 8a, a working part is provided with a keyed element 841. The keyed element 841 is dimensioned so that it may be inserted into the entry aperture 822. Other regions of the working part are excluded for clarity.

As shown in figure 8a, the slot 820 defines substantially a T cross section, and the keyed element 841 is shaped correspondingly. This shape achieves the objective of ensuring that the keyed element can only enter or exit the slot through the entry aperture 822, and that once it is slid from the entry aperture to fully engage the slot 822, no force in any direction on the working element with respect to the interface part will separate the working element secondary element from the interface part. The skilled person will recognise that the keyed element and corresponding slot may have any form complying with the general requirement that it widens from the distal end towards the proximal end. As such, it may form a wedge, dovetail or t section as described above. It may furthermore be circular, elliptical, rectangular, square, or any other form. The keyed element will generally constitute an extrusion of the chosen cross section from one side to the other. In certain embodiments, the keyed element may taper from one side to the other. Where this is the case the slot may taper from side to side correspondingly. Where this is the case, the slot may be defined as being deeper from side to side that the length from side to side of the corresponding keyed element. On this basis, the tapering walls of the keyed element will engage the sides of the keyed element before the end of the keyed element reached the lateral extremity of the slot. By this means, the slot will become progressively tighter as the keyed element is inserted, and a firm insertion without any play between the handle and secondary element may be achieved by pushing the keyed element fully into the slot.

The disposition of the first lateral slot 820 disposed along a second axis 811, the second axis in the plane of the first axis 810 and being at an angle θ of between 10 and 80 degrees to the longitudinal axis 810 means that the application of a force along the interface part through the working element will force the working element more deeply in to the slot to abut the end wall thereof, rather than tending to push the working element out of the slot.

Figure 8b shows an interface part for a modular tool in accordance with an embodiment in a second configuration.

The interface part of figure 8b comprises substantially the same elements as described with reference to figure 8a. As shown in figure 8a, the keyed element 841 has been inserted into the entry aperture 822. Other regions of the working part are excluded for clarity.

Figure 8c shows an interface part for a modular tool in accordance with an embodiment in a third configuration.

The interface part of figure 8c comprises substantially the same elements as described with reference to figures 8a and 8b. As shown in figure 8c, the keyed element 841 has been slid upward into the proximal extremity of the slot 820, away from the entry aperture 822. Since the keyed element 841 as shown widens from its base in correspondence to the widening of the slot 820, once slid upward in this manner the keyed element is trapped in the slot, the only possible path of exit being back through the entry aperture 822.

Optionally, the dimensions of the lateral slot may vary along the second axis so that the force required sliding a corresponding keyed element into the lateral slot increases as the corresponding keyed element of the tool progresses into the slot.

Meanwhile, a securing pin 850 is positioned at a proximal entrance 831 of securing pin channel 830, which terminates at the distal end of the interface part with the entry aperture 822, ready for insertion. The securing pin channel extends along a third axis, the third axis being at an angle in the plane of the first and second axes of between 90 degrees to the second axis and 30 degrees to the second axis in the proximal direction.

As shown, the securing pin 850 is substantially rectangular in cross section. In other embodiments, the securing pin may have any extruded form including a prism having any number of sides. Some or all sides may be curved. As such, the securing pin may be, for example, cylindrical, semicircular, triangular, square, rectangular and so on.

Since the slot 820 widens from the distal end 812 towards the proximal end 811, a correspondingly formed keyed element 841 may be slid into the slot laterally as shown in figure 8c, but once in position, will not be movable along the axis of the interface part, i.e. towards or away from the distal end of the interface part, but only back or forth along the axis of the slot.

Other regions of the working part are excluded for clarity.

Figure 8d shows an interface part for a modular tool in accordance with an embodiment in a fourth configuration.

The interface part of figure 8d comprises substantially the same elements as described with reference to figures 8a, 8b and 8c. As shown in figure 8d, the securing pin 850 has been inserted through the pin channel 830 so as to fill the entry aperture 822, thereby trapping the keyed element in the slot 820.

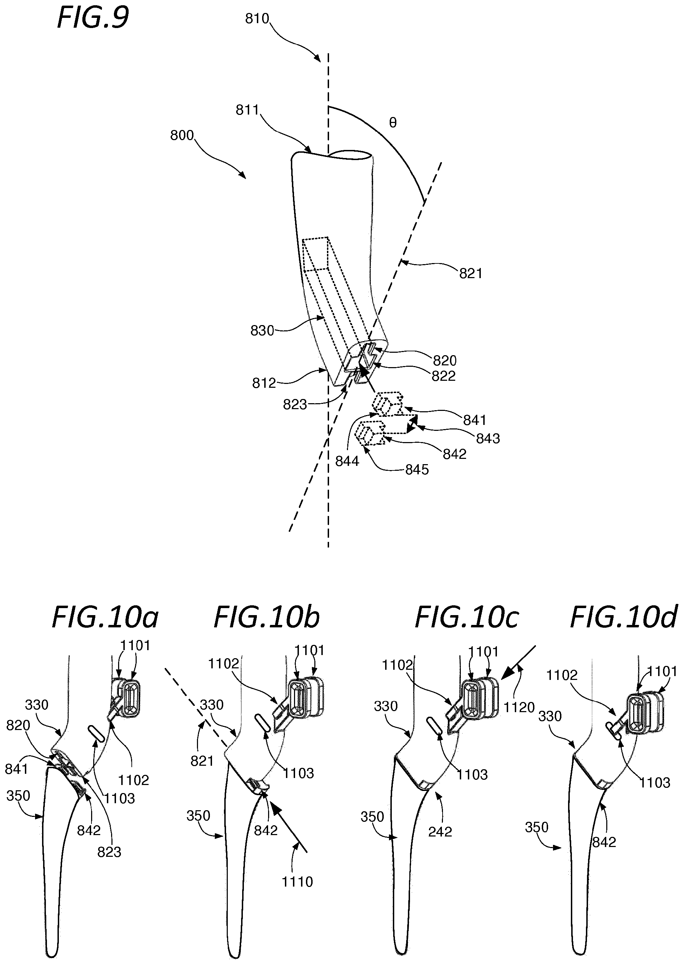

Figure 9 shows an interface element for a modular tool in accordance with a further embodiment.

The interface part of figure 9 comprises substantially the same elements as described with reference to figures 8a-8d above. As shown in figure 9, the interface part 800 comprises a second slot 823 parallel to the first slot 820. The second slot 823 may widen from top to bottom in a manner similar to the first slot 820. The second slot 823 may widen from top to bottom identically to the first slot 820. As shown, while the first slot widens to an entry aperture 822 at the distal end of the first slot 820, the second slot 823 opens laterally at the distal extremity of the interface part 800. As shown, the second slot is on the same axis 821 as the first slot. In other embodiments, the second slot may be situated in a further axis in parallel with the axis 821. Where the second slot does not open at the edge of the interface part as shown, it may open to a further respective entry aperture in the same way as the first slot. It will be appreciated that any number of slots, and corresponding keyed elements may be provided on this basis. Distributing slots across the distal surface of the interface part provides multiple connection points with the tool, and as such will tend to stabilize the connection between the tool and interface part.

It will be appreciated that there is provided a working part having features in correspondence to the tool holder of the present invention, and in particular to the releasable coupling thereof. In particular, there is provided a working element 800 for a modular tool, the working element having a distal end and a proximal end disposed on a longitudinal axis. The working element comprises a first keyed element 841 and a second keyed element 842 widening in a distal direction (so as not to be free to fall out of the slot in the distal direction once inserted), disposed along a second axis 810, and separated by a gap 843. By this means the working part may be secured in a corresponding tool holder 800 for example as described herein by inserting the first keyed element and said second keyed elements into corresponding slots 820, 823 defined in said tool holder 800, and inserting a securing pin 1102 through a securing pin channel 830 extending though said tool holder 800 and terminating in said gap 843 so as to trap a keyed element 841 inserted in said first slot.

Optionally, either or both of the keyed elements 841, 842 may be provided with a respective end plate 844, 845. Such end plates may serve to provide a solid end stop limiting and controlling the insertion of the keyed element into the slot. In particular, this approach may be used as well as or instead of the provision of the end wall and/or intermediate wall of the slot as described above.

Figures 10a, 10b, 10c and 10d illustrate the steps of insertion of a tool into an interface element as described above, and provide further details relating to the operation of the securing pin.

Figure 10a shows a tool and interface part in accordance with an embodiment in a first configuration.

As shown, the interface part 330 has a distal end 812 and a proximal end 811 disposed on a longitudinal axis 810. The interface part comprises a first lateral slot 820 disposed along a second axis 811, the second axis in the plane of the first axis 810 and being at an angle θ of between 10 and 80 degrees to the longitudinal axis 810. The slot widens from top to bottom, and further widens to an entry aperture 822 at the distal end of the first slot 820.

The interface part further comprises a securing pin channel 830 extending though the interface part and terminating in the entry aperture 822.

As shown in figure 10a, a tool 350 is provided with a first keyed element 841 and second keyed element 842, substantially as described above.

The interface part 330 of figure 10a meanwhile comprises a first slot 820 and second slot 823 substantially as described above.

The interface part comprises a securing pin channel (not shown) substantially as described above, into which a securing pin 850 is partially inserted. As shown, the securing pin further comprises optional opposing pinch plates 1101, which operate optional grasping members 1102 positioned either side of the body of the of the interface part and pivotally mounted on the securing pin 850. The grasping members 1102 are biased towards the securing pin 850. This biasing might be achieved by the provision of a spring or other resilient member. It may also be achieved by forming the grasping members and securing pin in a single piece, of a material which exhibits resilience when suitably formed.

As shown, the interface part 330 further comprises optional indentations 1103 on either side thereof, positioned either side of the pin channel entrance so that that when the securing pin 850 is fully inserted in the pin channel 830, each grasping member 1102 may engage a respective indentation 1103.

As such the interface part may comprise one or more features in the vicinity of the entrance of the securing pin channel adapted to engage a resilient member attached to the securing pin such that when inserted fully into the securing channel, the securing pin is resiliently retained in the securing channel.

As an alternative, features similar to indentations 1103 may be provided on the inside of the securing pin channel, and the securing pin provided with protuberances adapted to engage these features when the securing pin is inserted into the securing pin channel. The securing pin may be split partially along its lengths so as to provide two parallel portions resiliently joined at the distal end of the securing pin, so that the two portions may be squeezed together, for example by means of the pinch plates 1103 during insertion, such that when allowed to resume their unconstrained configuration, the protuberances engage the features.

It will be appreciated that other equivalent arrangement may be envisaged, for example with indentations on the securing pin and protuberances on the inside or outside of the interface part, and so on.

Optionally, the securing pin and/or securing pin channel may have varying dimensions along the lengths, so as to restrict or inhibit the movement of the securing pin in the securing pin channel as it approaches a fully inserted position. Optionally, the securing pin and/or securing pin channel may have varying dimensions along the lengths, so as to restrict or inhibit the movement of the securing pin in the securing pin channel as it approaches a retracted position, so as to reduce the likelihood of the securing pin being accidentally removed from the securing pin channel, and possibly dropped or mislaid.

Generally, the interface part may provide one or more features in the vicinity of the entrance of the securing pin channel adapted to engage a resilient member attached to the securing pin such that when inserted fully into the securing channel, the securing pin is resiliently retained in the securing channel.

Figure 10b shows a tool and interface part in accordance with an embodiment in a second configuration.

As shown, the tool 350 and interface part 330 have been aligned so that the first keyed element 841 and second keyed element 842 are in the axis 821 of the first slot 820 and second slot 823. The tool 350 and interface part 330 are slightly offset in a distal direction so that the first keyed element 841 has been inserted into the entry aperture 822, while the second keyed element 842 is outside, but aligned with the second slot 823.

Once in this configuration, a force along the axis 821 as shown by arrow 1110 will cause the tool 350 and interface part 330 to fully engage.

Figure 10c shows a tool and interface part in accordance with an embodiment in a third configuration.

As shown, a force has been exerted along the axis 821 as shown by arrow 1110 in figure 10b, causing the tool 350 and interface part 330 to fully engage.

As such, the first keyed element 841 is fully inserted into the first slot 820, clearing the entry aperture 822, while the second keyed element 842 is fully inserted into the second slot 823.

Once in this configuration, the securing pin 1102 may be fully inserted into the pin channel (not shown) substantially as described above, by exertion of a force along the axis of the pin channel as represented by the arrow 1120.

Figure 10d shows a tool and interface part in accordance with an embodiment in a fourth configuration.

As shown, the securing pin has been fully inserted into the pin channel (not shown) substantially as described above.

As shown, the grasping members 1102 positioned either side of the body of the interface part and pivotally mounted on the securing pin have engaged the indentations 1103 on either side of the interface part, either by an automatic latching operation enabled by the form of the grasping members, or by manual operation of the pinch plates 1101 as described above.

In this configuration, the securing pin is positively engaged in the securing channel, and by securing the entry aperture 822 as described above ensures that no movement of the working part 1100 with respect to the interface part 330 is possible in any direction. Furthermore, the securing pin itself is locked in the securing channel by the grasping members so that without the deliberate action of a user to disengage the grasping members and remove the securing pin, the working element and interface part are indissociable.

As such, the sequence of configurations 10a, 10b, 10c and 10d represent a series of simple manipulations which may be undertaken by a user. For example, the user may grip the interface part in one hand with their thumb resting on the end of the securing pin, while gripping the working element in the other hand. The user may then bring the interface part and working element together, and engage the keyed elements of the working element in the corresponding slots of the interface part, and slide the working element to the end of the available travel in the slots, before pushing the securing pin into position with the thumb so as to lock the working element in place.

To further facilitate these actions, the elements may be knurled, grooved, provided with a non-slip coating or otherwise treated to improve the users grip thereon. In particular the securing pin may be knurled, grooved, provided with a non-slip coating or provided with a loop, hook, or other formation adapted to engage the users thumb such that the securing pin may be both pushed forward to block the entry aperture, and pulled back to unblock the entry aperture with the users thumb.

Meanwhile, when pinch plates 1101 are brought together, for example by being pinched together by the index finger and thumb of a user, the grasping members are separated, so that the securing pin may be pulled back out of the pin channel, freeing the tool 350 with respect to the interface part 330 and making it possible for the two parts to be separated in the reverse of the sequence of figures 10c, 10b and 10a.

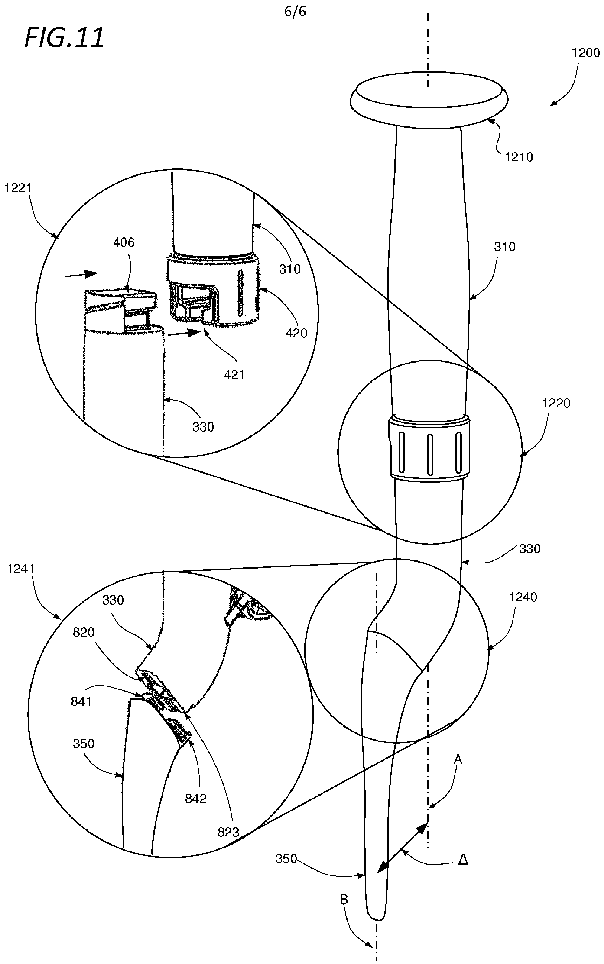

Figure 11 shows a tool in accordance with an embodiment.

As shown, there is provided a modular tool 1200 comprising a handle 310, and interface part 330 and an interface part 330 substantially as described above.

As described with respect to figure 3, the handle 310 is releasably coupled to the interface element 330 by a first releasable coupling 320, and the interface element 330 is releasably coupled to the tool element 350 by a second releasable coupling 340. In accordance with the embodiment of figure 11, the first coupling 320 is implemented by a coupling 1220, which as shown is a variant of that described above with reference to figures 8 to 10, presented in more detail in bubble 1221, and the second coupling 340 is implemented by a coupling 1240 a variant of that described above with reference to figures 8 to 10 presented in more detail in bubble 1241.

The proximal ends of the interface element 330 and the tool 350 are provided with keyed elements 406, 841, 842, so as to engage with the corresponding slot 421, 820, 823 as described above.

Any of the other variants or optional features presented above may be adopted, or not, in a modular tool along the lines of that of figure 11, as appropriate to the use case.

As shown, the handle further comprises an optional guard plate 1101 at the proximal end thereof. Such a guard plate may serve to protect the hand of a user when gripping the handle 310 from blows struck against the proximal end thereof with a hammer, mallet or the like, for example where the tool or tool 350 is a chisel, reamer or other such tool requiring a percussive application.

Thus, by assembling the three components (handle, interface element and tool) as described above, a tool may be assembled which due to the characteristics of the adopted coupling means exhibits mechanical rigidity comparable to that of a single unitary tool, with minimal risk of accidental dismantlement, whilst allowing a rapid assembly or dismantlement even with reduced dexterity, yet permitting the flexibility, reduction of weight and material usage achievable in a modular approach.

According to certain embodiments there is thus provided a modular surgical tool hip, for example for shoulder, knee or bone traumatology is provided comprising a handle, an interface part and a tool. The interface may serve to provide a selected linear and/or angular offset with regard to the respective axes of the handle and tool. The handle may be releasably coupled to the interface element by a threaded ring on a threaded cylindrical member of the handle. The handle may comprise a slot in which a keyed element of the interface may engage, to be trapped when the ring is turned about the cylindrical member. Meanwhile the interface may be releasably coupled to the tool by means of a keyed element of the tool which may be slid into a slot of the interface means, the slot being intersected by a securing pin channel passing through the body of the interface from the other side, such that when the securing pin is inserted the keyed member of the tool is trapped in the slot.

It will be understood that the configurations and/or approaches described herein are exemplary in nature, and that these specific embodiments or examples are not to be considered in a limiting sense, because numerous variations are possible. The specific routines or methods described herein may represent one or more of any number of processing strategies. As such, various acts illustrated and/or described may be performed in the sequence illustrated and/or described, in other sequences, in parallel, or omitted. Likewise, the order of the above-described processes may be changed.

The subject matter of the present disclosure includes all novel and non-obvious combinations and sub-combinations of the various processes, systems and configurations, and other features, functions, acts, and/or properties disclosed herein, as well as any and all equivalents thereof.

## Claims

1. A modular instrument for surgery of the hip, shoulder, knee or bone traumatology, said instrument comprising a handle part, an interface part and a tool element, said handle part being releasably coupled to said interface part and said interface part being releasably coupled to said tool part.

2. The modular instrument of claim 1 wherein said handle and said tool each has a respective primary axis, whereby the interface part is configured to establish a lateral offset between said axes.

3. The modular instrument of claim 1 or 2 wherein said handle and said tool each has a respective primary axis, whereby the interface part is configured to establish an angular offset between said axes.

4. The modular instrument of any preceding claim, said handle part comprising
a body having a distal end and a proximal end, said distal end providing a releasable coupling for an element of said modular tool, said releasable coupling comprising:
a cylindrical member provided with
a first external helical thread, and
a first lateral slot, said first slot opening on one periphery of said distal end of said body, said first slot widening from said distal end towards said proximal end,
said releasable coupling further comprising a threaded ring, the threads of said ring engaging said first external helical thread of said cylindrical member, said threaded ring being rotatable about said distal end of said member between an extended position in which said ring obstructs said first slot opening on one periphery of said distal end of said body, and a retracted position in which said ring leaves said first slot opening on one periphery of said distal end of said body unobstructed.

5. The modular instrument of any preceding claim wherein the angular displacement of said threaded ring when rotated between said extended position and said retracted position is between 89 and 181 degrees.

6. The modular instrument of any preceding claim wherein said threaded ring comprises an indentation at the distal edge thereof, said indentation being positioned on the circumference of said ring such that when said ring is in said retracted position said indentation is aligned with said first slot.

7. The modular instrument of any preceding claim wherein said interface part has a distal end and a proximal end disposed on a longitudinal axis, said interface part comprising a second lateral slot disposed along a second axis, said second axis in the plane of the first axis and being at an angle of between 10 and 80 degrees to said longitudinal axis, said second slot widening from top to bottom, said second slot widening to an entry aperture at the distal end of the second slot, said interface part further comprising a securing pin channel extending though said interface part and terminating in said entry aperture, such that a securing pin may be inserted through said pin channel so as to trap a keyed element inserted in the widened part of said second slot, whereby the interface part may be releasable coupled to said tool part by means of a keyed element of said tool engaged in said second slot and trapped by said securing pin.

8. The modular instrument of claim 7, wherein said securing pin channel extends along a third axis, said third axis being at an angle in the plane of the first and second axes of between 90 degrees to the second axis and 30 degrees to the second axis in the proximal direction.

9. The modular instrument of claim 7 or 8 wherein the dimensions of said lateral slot vary along said second axis so that the force required to slide a corresponding keyed element of said tool into said lateral slot increases as said corresponding keyed element of said tool progresses into said slot.

10. The modular instrument of any preceding claim wherein said modular tool comprises a surgical instrument.

11. The modular instrument of claim 10 wherein said modular instrument is for surgery of the hip, shoulder, knee or bone traumatology.

12. The modular instrument of any preceding claim wherein said modular instrument comprises a rasp or reamer.

13. The modular instrument of any preceding claim wherein at least a part thereof is composed of a synthetic material or a synthetic composite material.

14. The modular instrument of claim 12 wherein at least a part thereof is composed of a glass fiber reinforced polyarylamide.

15. A tool set comprising a handle according to any preceding claim, a tool element according to any preceding claim, and a plurality of interface elements according to any preceding claim, each said interface element defining a different combination of angular and/or linear off-sets with regard to the primary axes of the handle and tool when coupled to each respective said interface.
